# EUROPEAN PATENT SPECIFICATION

(11) **EP 2 832 802 B1**
(45) Date of publication and mention of the grant of the patent: **11.05.2016**
(21) Application number: 14162657.2
(22) Date of filing: 31.03.2014
(51) Int. Cl.: C09D 5/14, C01B 31/04, A61L 27/30, A61L 27/34, A61L 31/08, A61L 31/10, A61L 29/08, A61L 29/10

(54) **Method for production of coatings with antimicrobial properties**
Verfahren zur Herstellung von Beschichtungen mit antimikrobiellen Eigenschaften
Procédé pour la production de revetements possédant des propriétés antimicrobial

(30) Priority: 02.08.2013 LT 2013503
(43) Date of publication of application: 04.02.2015
(73) Proprietor: UAB Moksliniu Tyrimu Pletros Laboratorija, 01402 Vilnius (LT)
(72) Inventor: Barkauskas, Jurgis, 06310 Vilnius (LT)
(74) Representative: Draugeliene, Virgina Adolfina

(56) References cited:
- EP-A1- 2 567 938
- DE-C- 565 557
- YU YAN ET AL: "Ultrafast room temperature wiping-rubbing fabrication of graphene nanosheets as flexible transparent conductive films with high surface stability", APPLIED PHYSICS LETTERS, AMERICAN INSTITUTE OF PHYSICS, US, vol. 101, no. 2, 9 July 2012 (2012-07-09), pages 23119-23119, XP012163889, ISSN: 0003-6951, DOI: 10.1063/1.4736576 [retrieved on 2012-07-13]
- SHICHAO ZHAO ET AL: "Paper;Photochemical oxidation of CVD-grown single layer graphene;Photochemical oxidation of CVD-grown single layer graphene", NANOTECHNOLOGY, IOP, BRISTOL, GB, vol. 23, no. 35, 8 August 2012 (2012-08-08), page 355703, XP020228659, ISSN: 0957-4484, DOI: 10.1088/0957-4484/23/35/355703
- HUMMERS W S ET AL: "Preparation of graphite oxide", JOURNAL OF THE AMERICAN CHEMICAL SOCIETY, AMERICAN CHEMICAL SOCIETY, vol. 80, no. 6, 20 March 1958 (1958-03-20) , page 1339, XP008127696, ISSN: 0002-7863
- WENBING HU ET AL: "Graphene-Based Antibacterial Paper", ACS NANO, vol. 4, no. 7, 27 July 2010 (2010-07-27), pages 4317-4323, XP055155239, ISSN: 1936-0851, DOI: 10.1021/nn101097v
- DATABASE WPI Week 201146 Thomson Scientific, London, GB; AN 2011-E95422 XP002733404, & CN 101 990 899 A (SHANGHAI INST APPLIED PHYSICS CHINESE AC) 30 March 2011 (2011-03-30)

## Description

The present invention relates to the methods and devices of production of coatings with antimicrobial properties, in particular, to a method and device of the production of coating of oxidized graphene on a polypropylene film, where coating obtained by this way obtain antimicrobial properties and can be used in food industry, medicine, agriculture, domestic appliances and elsewhere.

Antibacterial properties of graphene are described in C. M. Santos, J. Mangadlao, F. Ahmed, A. Leon, R. C. Advincula, D. F. Rodrigues. Graphene nanocomposite for biomedical applications: fabrication, antimicrobial and cytotoxic investigations. Nanotechnology 23 (2012) 395101, where antimicrobial properties of graphene coatings and biocompatibility with human tissues were investigated and their antibacterial activity and good biocompatibility were ascertained. Dispersions and organic solvents are used in the process of coating preparation; therefore their production technology is complicated. The method of electrodeposition, which rate is slow, is used for the production.

In International Patent Application WO 2013/050554 A1 is described the method of formation of the coatings with antimicrobial properties by immobilization of e.g. nanoparticles of graphite oxide on the functionalized surfaces that are able to connect biologically active molecules (carbohydrates, nanoparticles of graphite oxide). Technology of the coating preparation using this method is complicated, since it requires functionalized surfaces of special preparation.

International Patent Application WO 2012/075294 A2, 2010 12 01 describes the preparation method of coatings composed of graphene (as well as carbon nanotubes) from nanocomposite dispersions; the coatings may include electroconductive polymers. Dispersions used for the coating manufacture are instable, their preparation incur additional cost.

This invention is intended to simplify the methods of production of coatings with antimicrobial properties.

According to the present invention a method of production of coating with antimicrobial properties comprises even distributing a graphite powder on a surface of a polypropylene film and mechanical rubbing the graphite powder onto the surface of the polypropylene film to form a thin layer of graphene, washing with a water of the polypropylene film with the formed thin layer of graphene, oxidation of the formed thin layer of graphene to a graphene oxide and washing with distilled water and drying of the polypropylene film with the formed thin layer of the graphene oxide. Mechanical rubbing of the graphite powder onto the surface may take about 30 seconds.The graphene layer may be oxidized by immersion the polypropylene film with the formed thin layer of graphene into a solution obtained dissolving of 0.5 g NaNO₃ and 3.0 g KMnO₄ in 23.0 ml concentrated H₂SO₄ for about 30 seconds.

Advantage offered by the invention is that proposed method of the production of coating with antimicrobial properties are simple and do not require sophisticated equipment, special processes and materials.

This invention is explained in detail in drawing showing the device for the mechanical covering of polypropylene film with graphene layer.

According to the invention, the method of production of coating with antimicrobial properties includes the following sequence of operations: graphite powder is evenly distributed and mechanical rubbed onto a surface of a polypropylene film. During the mechanical rubbing, which lasts about 30 seconds, graphene layer is formed on the surface of the film. Further, the polypropylene film with the formed graphene layer is washed with water and graphene layer is oxidized to grapheme oxide. A solution prepared by mixing of 23 ml conc. H₂SO₄ with 0.5 g NaNO₃ and 3.0 g KMnO₄ is used for the oxidation. The polypropylene film with graphene layer is immersed into the solution for 30 seconds. After the stage of oxidation the polypropylene film with the thin layer of graphene oxide is washed with water and dried.

The proposed method is realized by a device shown in drawing, which comprises a cover 1, which is designed in the form of cylinder and is used for the close compressing of the polypropylene film 2 to a smooth base. The polypropylene film 2 may be e.g. in the form of circle about 5 cm in diameter. The cover 1 is provided with a hole for the adding of a graphite powder 3. A brush 4 is inserted into the cover 1 with the capability to move and rotate for the even covering and mechanical rubbing the graphite powder 3 on the surface of the polypropylene film 2. During the rotation the brush 4 mechanically covers the surface of the polypropylene film 2 with a the graphite powder 3 and rubs them onto the surface to form the thin layer of graphene. The brush 4 is connected to a drive 6 via a flexible connection. After this procedure the cover 1 is replaced and the polypropylene film 2 with the thin layer of graphene is washed under the stream of cold water. Later, the oxidation of graphene is performed via the method described above. The film prepared by this way has anti-microbial properties and can be used in medicine, food industry, building works, etc.

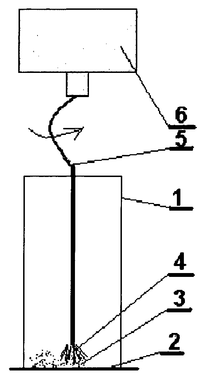

## Claims

1. Method of production of coating with antimicrobial properties comprising applying of a thin layer of graphene to a polypropylene film (2), **characterized by** the steps of:
- even distributing a graphite powder (3) on a surface of the polypropylene film (2) and mechanical rubbing the graphite powder (3) on the surface of the polypropylene film (2) to form the thin layer of graphene,
- washing with a water of the polypropylene film (2) with the formed thin layer of graphene
- oxidation of the formed thin layer of graphene to a thin layer of graphene oxide,
- washing with distilled water and drying of the polypropylene film (2) with the thin layer of graphene oxide

2. Method according to claim 1, **characterized in that** the mechanical rubbing of the graphite powder on the surface takes 30 seconds.

3. Method according to claim 1 or claim 2, **characterized in that** the graphene layer is oxidized by immersion the film (2) with the formed thin layer of graphene into a solution obtained dissolving of 0.5 g NaNO₃ and 3.0 g KMnO₄ in 23.0 ml concentrated H₂SO₄ for 30 seconds.

## Patentansprüche

1. Verfahren zur Herstellung eine Beschichtung mit antibakteriellen Eigenschaften in Form einer dünnen Graphenschicht auf einer Polypropylenfolie (2), **gekennzeichnet durch die Schritte::**
- Gleichmäßiges Verteilen von Graphitpulver (3) auf der Oberfläche der Polypropylenfolie (2) und mechanisches Verreiben des Graphitpulvers (3) auf der Oberfläche der Polypropylenfolie (2), um eine dünne Graphenschicht zu bilden;
- Abwaschen der Polypropylenfolie (2) mit der dünnen Graphenschicht mit Wasser;
- Oxidieren der gebildeten dünnen Graphenschicht zur einer dünnen Graphenoxidschicht;
- Waschen der Polypropylenfolie (2) mit der dünnen Graphenoxidschicht mit destilliertem Wasser und anschließendes Trocknen.

2. Verfahren nach Anspruch1, **dadurch gekennzeichnet, dass** das mechanische Verreiben des Graphitpulvers auf der Oberfläche 30 Sekunden in Anspruch nimmt.

3. Verfahren nach Anspruch 1 oder 2, **dadurch gekennzeichnet, dass** die Graphenschicht durch 30 Sekunden Eintauchen der Folie (2) mit der dünnen Graphenschicht in eine Lösung aus 0,5 g NaNO₃ und 3,0 g KMO4 in 23,0 ml konzentrierter H₂SO₄ oxidiert wird.

## Revendications

1. Procédé de production d'un film avec des propriétés antimicrobiennes comprenant l'application d'une mince couche de graphène à un film de polypropylène (2), **caractérisé par les étapes consistant à :**
- distribution de manière égale d'une poudre de graphite (3) sur une surface du film de polypropylène (2) et le frottement mécanique de la poudre de graphite (3) sur la surface du film de polypropylène (2) pour former la mince couche de graphène,
- lavage avec de l'eau du film de polypropylène (2) avec la couche mince formée de graphène,
- oxydation de la couche mince formée du graphène par l'obtention d'une couche mince d'oxide de graphène,
- lavage à l'eau distillée et séchage du film de polypropylène (2) avec la couche mince d'oxyde de graphène.

2. Procédé selon la revendication 1 **caractérisé en ce que** le frottement mécanique de la poudre de graphite sur la surface prenne 30 secondes.

3. Procédé selon la revendication 1 ou la revendication 2, **caractérisé en ce que** la couche de graphene est oxydé par l'immersion du film (2) formée avec la couche mince graphène dans une solution obtenu par la dissolution de 0,5 g de NaNO3 et 3,0 g de KMnO4 dans 23.0 ml de H2SO4 concentré pendant 30 secondes.
